# EUROPEAN PATENT SPECIFICATION

(11) **EP 3 419 686 B1**
(45) Date of publication and mention of the grant of the patent: **23.09.2020**
(21) Application number: 17710434.6
(22) Date of filing: 16.02.2017
(51) Int. Cl.: A61M 1/00

(54) **MANUALLY ACTIVATED NEGATIVE PRESSURE THERAPY SYSTEM WITH INTEGRATED AUDIBLE FEEDBACK**
HANDBETÄTIGTES UNTERDRUCKTHERAPIESYSTEM MIT INTEGRIERTEM HÖRBAREM FEEDBACK
SYSTÈME DE THÉRAPIE PAR PRESSION NÉGATIVE ACTIVÉ MANUELLEMENT À RÉPONSE AUDIBLE INTÉGRÉE

(30) Priority: 22.02.2016 US 201662298052 P
(43) Date of publication of application: 02.01.2019
(73) Proprietor: KCI Licensing, Inc., San Antonio, TX 78265-9508 (US)
(72) Inventor: LOCKE, Christopher, Brian, Bournemouth BH9 3SD (GB)
(74) Representative: Simmons & Simmons
(86) International application number: PCT/US2017/018129
(87) International publication number: WO 2017/146986

(56) References cited:
- WO-A1-2013/078214
- US-A1- 2007 265 586
- US-A1- 2015 018 784
- US-A1- 2015 094 673
- US-A1- 2015 094 674

## Description

### TECHNICAL FIELD

The invention set forth in the appended claims relates generally to tissue treatment systems and more particularly, but without limitation, to providing feedback in negative-pressure therapy systems.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

While the clinical benefits of negative-pressure therapy are widely known, improvements to therapy systems, components, and processes may benefit healthcare providers and patients.

US2007/0265586 discloses a wound therapy device including a housing for covering a wound, a liquid collector, and a vacuum source with a pressure sensor.

US2015/0094674 and US2015/0094673 disclose a system for providing feedback for reduced-pressure treatment of a tissue site using a pressure regulator.

WO2013/078214 discloses a system for treating a plurality of tissue sites using a multi-port therapy unit. A controller monitors the pressure delivered to each tissue site.

US2015/018784 discloses a manually actuated reduced pressure source.

### BRIEF SUMMARY

There is provided a manually actuated pump, for providing reduced pressure treatment comprising: a housing defining a regulated chamber and a charging chamber in fluid communication with the regulated chamber, wherein the charging chamber is configured to be supplied with a reduced pressure, and wherein the regulated chamber is configured to regulate the reduced pressure to a regulated reduced pressure; and a feedback module comprising a controller, an output device, and a pressure sensor in fluid communication with the charging chamber, wherein the pressure sensor is configured to monitor reduced pressure within the charging chamber and provide a pressure signal to the controller, and the controller is configured to provide a feedback signal to the output device for generating at least one of an audible warning and a visual warning based on the pressure signal, wherein the controller is configured to activate at least one of an audio output device and a visual output device based on a comparison between the pressure signal and a recharge threshold.

A selection of optional features are set out in the dependent claims.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

FIG. 1 is a perspective view of a reduced pressure treatment system according to an exemplary embodiment;
FIG. 2 is a cross-sectional front view of a dressing of FIG. 1 taken at line 2-2;
FIG. 3 is a side perspective view of a reduced pressure treatment apparatus according to an exemplary embodiment;
FIG. 4 is a front view of the reduced pressure treatment apparatus of FIG. 3;
FIG. 5 is an exploded side perspective view of the reduced pressure treatment apparatus of FIG. 3;
FIG. 6 is an exploded rear perspective view of the reduced pressure treatment apparatus of FIG. 3;
FIG. 7 is a cross-sectional view of the reduced pressure treatment apparatus in a first position taken at line 11-11 of FIG. 4;
FIG. 8 is a top-rear perspective view of a piston of the reduced pressure treatment apparatus of FIG. 3;
FIG. 9 is a bottom-rear perspective view of the piston of FIG. 8;
FIG. 10 is a top-rear perspective view of a seal of the reduced pressure treatment apparatus of FIG. 3;
FIG. 11 is a bottom-rear perspective view of the seal of FIG. 10;
FIG. 12 is a top-rear perspective view of a portion of a second barrel of the reduced pressure treatment apparatus of FIG. 3;
FIG. 13 is a bottom-rear perspective view of the portion of the second barrel of FIG. 12;
FIG. 14 is a cross-sectional view of the reduced pressure treatment apparatus in a second position taken along line 11-11 of FIG. 4;
FIG. 15 is an enlarged cross-sectional view of the reduced pressure treatment apparatus of FIG. 14, the reduced pressure treatment apparatus having a valve body shown in a closed position;
FIG. 16 is an enlarged cross-sectional view of the reduced pressure treatment apparatus of FIG. 15 with the valve body shown in an open position;
FIG. 17 is a sectional exploded view of the closed end of a charging chamber including a housing extension and feedback module according to an exemplary embodiment;
FIG. 18 is a functional block diagram of a feedback module according to an exemplary embodiment; and
FIG. 19 is a cross-sectional view of an example alternative reduced pressure treatment apparatus in a first position taken at line 11-11 of FIG. 4.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

Reduced pressure treatment systems are often applied to large, highly exudating wounds present on patients undergoing acute or chronic care, as well as other severe wounds that are not readily susceptible to healing without application of reduced pressure. Low-severity wounds that are smaller and produce less exudate have generally been treated using advanced dressings instead of reduced pressure treatment. Improvements in wound healing, however, may be obtained by using reduced pressure treatment, even with smaller and less severe wounds.

Eliminating power requirements can increase mobility and generally reduce cost, which may be particularly beneficial for patients with low-acuity wounds. For example, a manually-actuated or manually-charged pump can be used as a source of reduced pressure instead of an electrically-powered pump. A manually-actuated pump also works well for wound treatment where there is no hospital infrastructure accessible to the patient or a limited supply of medical equipment.

Reduced pressure treatment systems and methods as described herein can provide a manually-actuated pump that incorporate an electronic feedback module, which can substantially reduce power requirements while still providing important signals to an operator. The feedback module may include components such as a pressure sensor (e.g., a pressure transducer), a controller or processor, memory, one or more visual indicators (e.g., light emitting diodes, or LEDs), one or more audio indicators (e.g., one or more speakers), a power source (e.g., a battery), and/or other components for providing various functions as described herein. The feedback module may include a printed circuit board (PCB) with the components of the feedback module mounted thereon. The functions provided by the feedback module may include, but are not limited to: monitoring pressure within a charging chamber of the pump and providing a visual and/or audio indication of the pressure; monitoring usage of the pump to determine a remaining usable period of the pump and providing a visual and/or audio indication thereof; monitoring the pressure within the charging chamber to determine whether the pressure is greater than an over-pressure threshold or is less than an under-pressure threshold and providing a visual and/or audio indication thereof; providing an indication of remaining battery life; storing data related to the operation of the pump in memory and selectively providing the data to a user; and/or further functions as described herein.

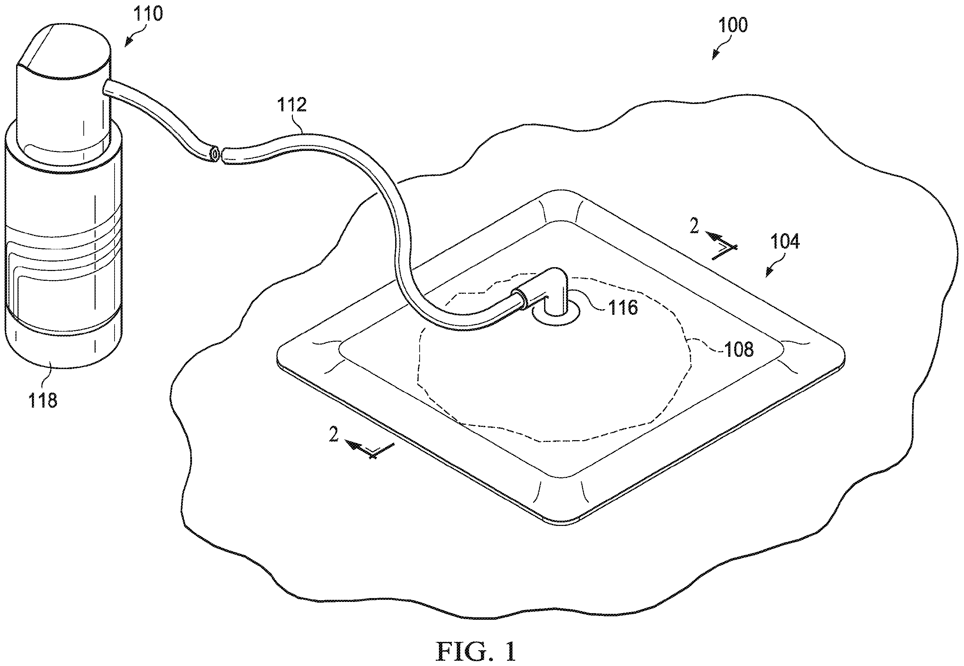

Referring to FIG. 1, a reduced pressure treatment system 100 according to an exemplary embodiment includes a reduced pressure dressing 104 positioned at a tissue site 108. The reduced pressure dressing 104 may be fluidly connected to a reduced-pressure source 110 by a conduit 112. The conduit 112 may fluidly communicate with the reduced pressure dressing 104 through a tubing adapter 116. In the exemplary embodiment of FIG. 1, the reduced-pressure source 110 is a manually-actuated pump. In other exemplary embodiments, the reduced-pressure source 110 may include pressure regulation capabilities and may initially be charged or re-charged to a selected reduced pressure by an external reduced-pressure source, such as an electrically driven pump or wall-suction source, for example. In still other embodiments, the reduced-pressure source 110 may be charged to the selected reduced pressure by a wall-suction source such as those available in hospitals and other medical facilities.

In general, components of the reduced-pressure treatment system 100 may be coupled directly or indirectly. For example, the reduced-pressure source 110 may be directly coupled to the conduit 112 and indirectly coupled to the reduced pressure dressing 104 through the conduit 112. In other embodiments, the reduced-pressure source 110 may be directly coupled to a canister (not shown) and indirectly coupled to the reduced pressure dressing 104 through the canister. Components may be fluidly coupled to each other to provide a path for transferring fluids (i.e., liquid and/or gas) between the components. In some embodiments, components may be fluidly coupled with a tube, such as the conduit 112, for example. A "tube," as used herein, broadly refers to a tube, pipe, hose, conduit, or other structure with one or more lumina adapted to convey fluids between two ends. Typically, a tube is an elongated, cylindrical structure with some flexibility, but the geometry and rigidity may vary. In some embodiments, components may additionally or alternatively be coupled by virtue of physical proximity, being integral to a single structure, or being formed from the same piece of material. Coupling may also include mechanical, thermal, electrical, or chemical coupling (such as a chemical bond) in some contexts.

The reduced-pressure source 110 may be housed within or used in conjunction with a reduced pressure treatment unit (not shown), which may also contain sensors, processing units, alarm indicators, memory, databases, software, display units, and user interfaces that further facilitate the application of reduced pressure treatment to the tissue site 108. In one example, a sensor or switch (not shown) may be disposed at or near the reduced-pressure source 110 to determine a source pressure generated by the reduced-pressure source 110. The sensor may communicate with a processing unit that monitors and controls the reduced pressure that is delivered by the reduced-pressure source 110. Delivery of reduced pressure to the reduced pressure dressing 104 and the tissue site 108 encourages new tissue growth by maintaining drainage of exudate from the tissue site 108, increasing blood flow to tissues surrounding the tissue site 108, and creating microstrain at the tissue site 108.

The reduced-pressure source 110 includes an electronic feedback module as described below in more detail. For example only, the feedback module may be mounted upon a PCB enclosed within a housing extension 118 of the reduced-pressure source 110.

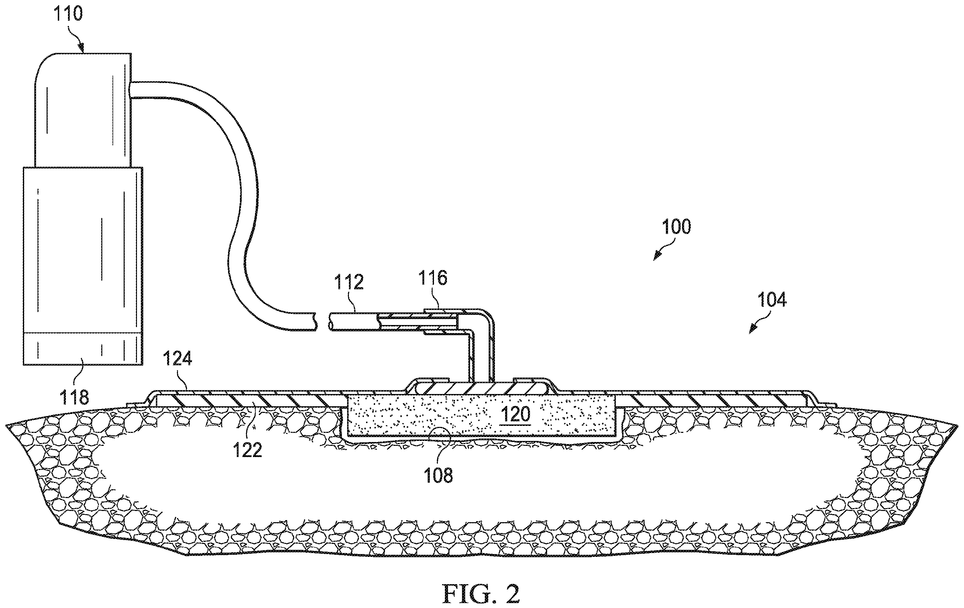

FIG. 2 is a sectional view, illustrating additional details of the reduced pressure dressing 104. The reduced pressure dressing 104 includes a distribution manifold 120 adapted to be positioned at the tissue site 108, and a seal layer 122 adapted to seal the reduced pressure dressing 104 to tissue proximate the tissue site 108. A cover 124 is positioned over the distribution manifold 120 and the seal layer 122 to maintain reduced pressure beneath the cover 124 at the tissue site 108. The cover 124 may extend beyond a perimeter of the tissue site 108 and may include an adhesive or bonding agent on the cover 124 to secure the cover 124 to tissue adjacent the tissue site 108. In some embodiments, the adhesive disposed on the cover 124 may be used in lieu of the seal layer 122. In other embodiments, the seal layer 122 may be used in conjunction with the adhesive of the cover 124 to improve sealing of the cover 124 at the tissue site 108. In another embodiment, the seal layer 122 may be used in lieu of adhesive disposed on the cover 124.

The cover 124 is an example of a sealing member and may also be referred to as a drape. A sealing member may be constructed from a material that can provide a fluid seal between two components or two environments, such as between a therapeutic environment and a local external environment. A sealing member may be, for example, an impermeable or semi-permeable, elastomeric material that can provide a seal adequate to maintain a reduced pressure at a tissue site for a given reduced-pressure source. For semi-permeable materials, the permeability generally should be low enough that a desired reduced pressure may be maintained. An attachment device may be used to attach a sealing member to an attachment surface, such as undamaged epidermis, a gasket, or another sealing member. The attachment device may take many forms. For example, an attachment device may be a medically-acceptable, pressure-sensitive adhesive that extends about a periphery of, a portion of, or an entire sealing member. Other example embodiments of an attachment device may include a double-sided tape, paste, hydrocolloid, hydrogel, silicone gel, organogel, or an acrylic adhesive.

The distribution manifold 120 can be generally adapted to contact a tissue site. The distribution manifold 120 may be partially or fully in contact with the tissue site. If the tissue site is a wound, for example, the distribution manifold 120 may partially or completely fill the wound, or may be placed over the wound. Although the distribution manifold 120 illustrated in FIG. 2 has a rectangular cross-section, the distribution manifold 120 may take many forms, and may have many sizes, shapes, or thicknesses depending on a variety of factors, such as the type of treatment being implemented or the nature and size of a tissue site. For example, the size and shape of the distribution manifold 120 may be adapted to the contours of deep and irregular shaped tissue sites.

More generally, a manifold is a substance or structure adapted to reduce pressure across a tissue site. In some embodiments, though, a manifold may also facilitate removing or delivering fluids to a tissue site, if the fluid path is reversed or a secondary fluid path is provided, for example. A manifold may include flow channels or pathways that distribute fluids provided to and removed from a tissue site around the manifold. In one exemplary embodiment, the flow channels or pathways may be interconnected to improve distribution of fluids provided to or removed from a tissue site. For example, cellular foam, open-cell foam, porous tissue collections, and other porous material such as gauze or felted mat generally include structural elements arranged to form flow channels. Liquids, gels, and other foams may also include or be cured to include flow channels.

In one exemplary embodiment, the distribution manifold 120 may be a porous foam material having interconnected cells or pores adapted to uniformly (or quasi-uniformly) distribute reduced pressure to a tissue site. The foam material may be either hydrophobic or hydrophilic. In one non-limiting example, the distribution manifold 120 can be an open-cell, reticulated polyurethane foam such as GranuFoam® dressing available from Kinetic Concepts, Inc. of San Antonio, Texas.

In an example in which the distribution manifold 120 may be made from a hydrophilic material, the distribution manifold 120 may also wick fluid away from a tissue site while continuing to distribute reduced pressure to the tissue site. The wicking properties of the distribution manifold 120 may draw fluid away from a tissue site by capillary flow or other wicking mechanisms. An example of a hydrophilic foam is a polyvinyl alcohol, open-cell foam such as V.A.C. WhiteFoam® dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

The distribution manifold 120 may further promote granulation at the tissue site 108 if a reduced pressure is applied through the reduced pressure dressing 104. For example, any or all of the surfaces of the distribution manifold 120 may have an uneven, coarse, or jagged profile that causes microstrains and stresses at the tissue site 108 if reduced pressure is applied through the distribution manifold 120. These microstrains and stresses have been shown to increase new tissue growth.

In some embodiments, the distribution manifold 120 may be constructed from bioresorbable materials that do not have to be removed from a patient's body following use of the reduced pressure dressing 104. Suitable bioresorbable materials may include, without limitation, a polymeric blend of polylactic acid (PLA) and polyglycolic acid (PGA). The polymeric blend may also include without limitation polycarbonates, polyfumarates, and capralactones. The distribution manifold 120 may further serve as a scaffold for new cell-growth, or a scaffold material may be used in conjunction with the distribution manifold 120 to promote cell-growth. A scaffold is a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

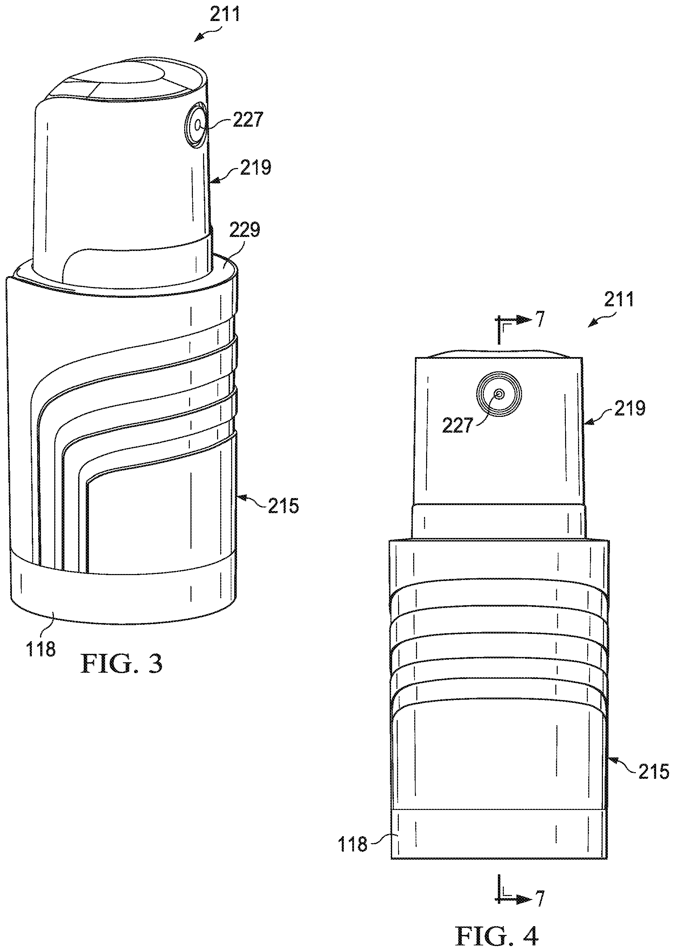

FIG. 3 is a perspective view and FIG. 4 is a front elevation view, illustrating additional details of a reduced-pressure source 211. The reduced-pressure source 211 may also be referred to as a reduced pressure treatment apparatus, a manually-actuated pump, or a pump. The reduced-pressure source 211 may have an outer barrel, such as a first barrel 215, and an inner barrel, such as a second barrel 219. While the first barrel 215 and the second barrel 219 are illustrated as having substantially cylindrical shapes, the shapes of the first barrel 215 and the second barrel 219 could be other shapes that permit operation of the device. An outlet port 227 may be provided on the second barrel 219 and may be adapted for fluid communication with a delivery tube or other conduit, which may be similar to the conduit 112, such that reduced pressure generated by the reduced-pressure source 211 may be delivered to the tissue site, such as the tissue site 108. The reduced-pressure source 211 may further include a barrel ring 229. The barrel ring 229 can be positioned at an open end of the first barrel 215 to circumscribe the second barrel 219. The barrel ring 229 can eliminate gaps between the first barrel 215 and the second barrel 219 at the open end of the first barrel 215.

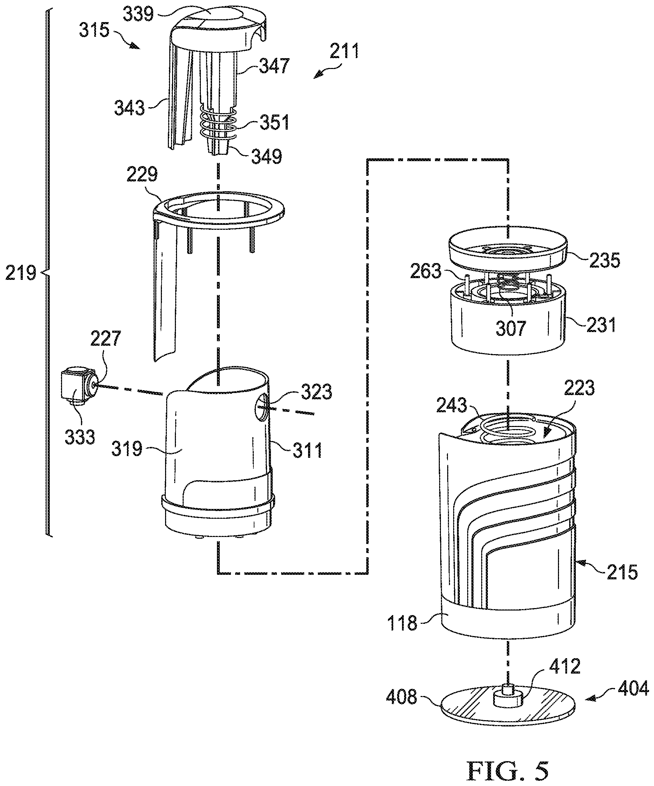

FIG. 5 is an exploded view, illustrating additional details of the reduced-pressure source 211. As shown in FIG. 5, the first barrel 215 includes a passage 223 having the open end of the first barrel 215. The passage 223 may be defined by a substantially cylindrical wall. The passage 223 slidingly receives the second barrel 219 through the open end of the first barrel 215, and the second barrel 219 is movable between an extended position and a compressed position. The housing extension 118 may be coupled to or integral with the first barrel 215. A feedback module 404 may be supported by or enclosed within the housing extension 118. The feedback module 404 may include a PCB 408 and a pressure sensor 412, for example, and may also include other components as described below in more detail that may be mounted on the PCB 408.

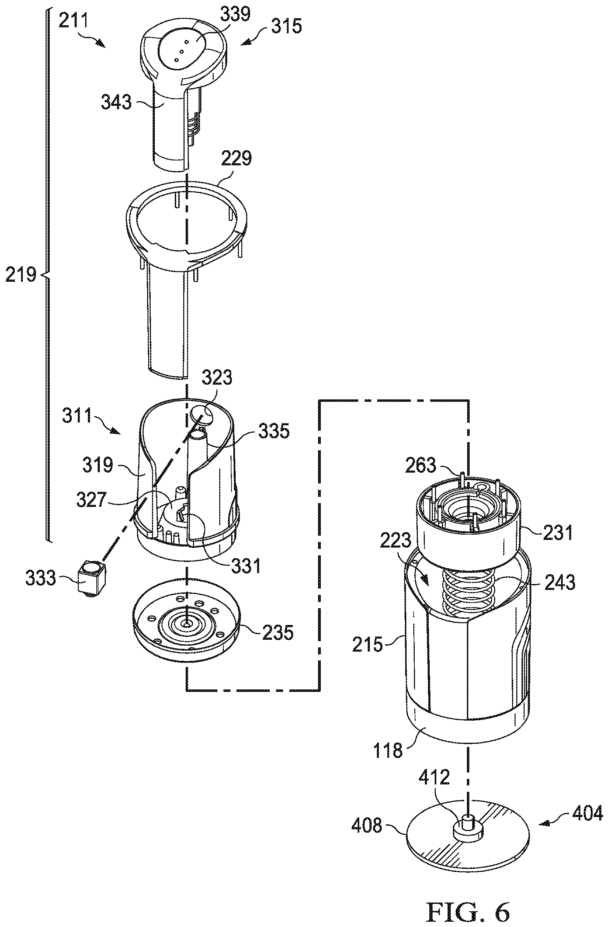

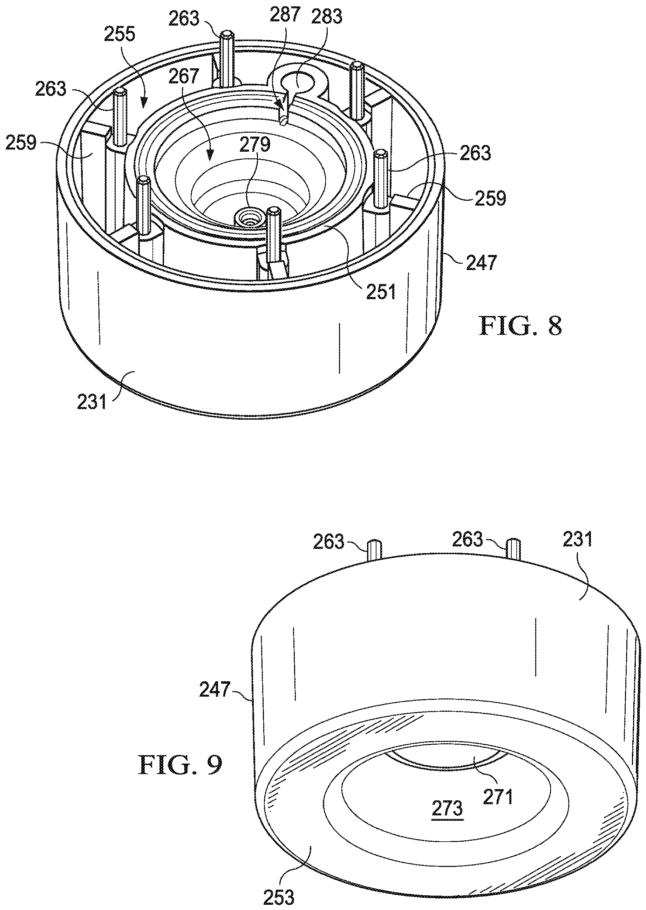

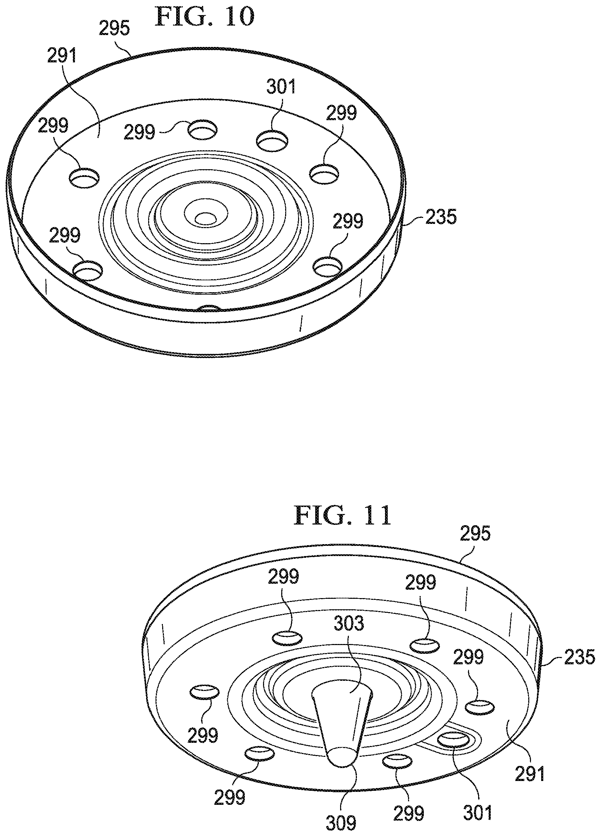

FIG. 6 is another exploded view, illustrating additional details that may be associated with the reduced-pressure source 211 in some embodiments. As shown in FIGS. 9 and 10, the reduced-pressure source 211 further includes a piston 231 and a seal 235. If the reduced-pressure source 211 is assembled, the piston 231 and the seal 235 are slidingly received within the passage 223 of the first barrel 215. Both the piston 231 and the seal 235 are positioned in the passage 223 between the second barrel 219 and an end of the first barrel 215 opposite the open end of the first barrel 215, the seal 235 being positioned between the second barrel 219 and the piston 231.

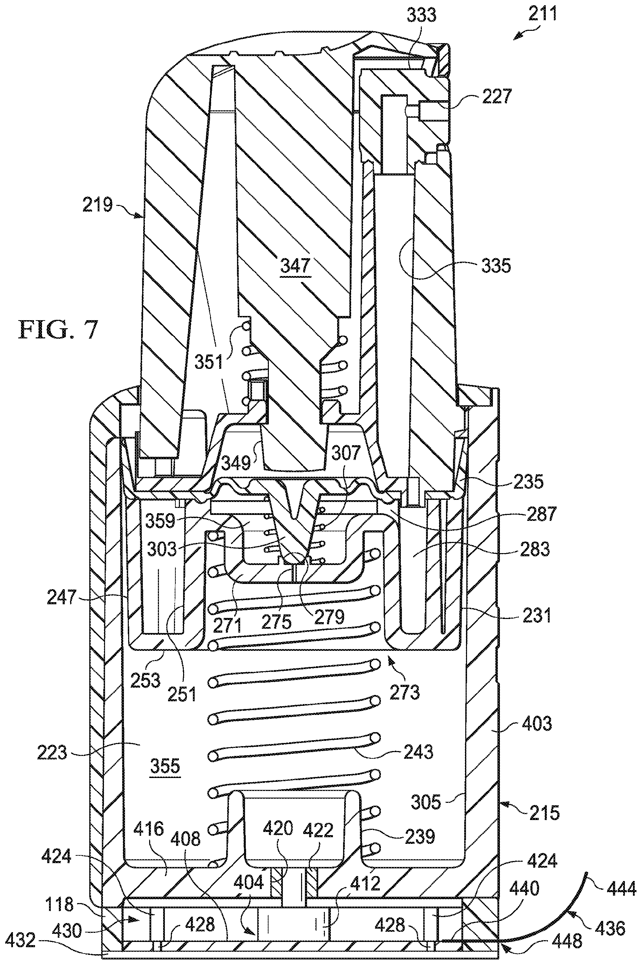

FIG. 7 is a sectional view, illustrating additional details that may be associated with some example embodiments of the reduced-pressure source 211. The first barrel 215 of FIG. 7 includes a closed end opposite the open end of the first barrel 215. The closed end of the first barrel 215 may be formed by an outer wall 416 having a peripheral portion joined to a cylindrical wall 403 forming the passage 223. The first barrel 215 includes a protrusion 239 extending from the outer wall 416 of the first barrel 215 into the passage 223. A piston spring 243 or other biasing member may be positioned within the passage 223, and the protrusion 239 receives an end of the piston spring 243. The protrusion 239 can reduce lateral movement of the piston spring 243 within the passage 223. The piston 231 may receive an opposite end of the piston spring 243. The piston spring 243 biases the piston 231, the seal 235, and the second barrel 219 toward the extended position illustrated in FIG. 7.

FIG. 8 and FIG. 9 are perspective views illustrating additional details of the piston 231. The piston 231 includes an outer wall 247 and an inner wall 251 joined by an outer floor 253. An annulus 255 is defined between the outer wall 247 and the inner wall 251, and a plurality of radial supports 259 are positioned between the outer wall 247 and the inner wall 251 in the annulus 255. The radial supports 259 provide additional rigidity to the piston 231. The presence of the annulus 255 as well as the sizes and spacing of the radial supports 259 within the annulus 255 reduces the weight of the piston 231 as compared to a single-wall piston that includes no annulus. However, it should be apparent that either piston design would be suitable for the reduced-pressure source described herein.

A plurality of guides 263 are disposed on the piston 231, and in some embodiments, a guide 263 can be disposed on each radial support 259. As described in more detail herein, the guides 263 serve to align the piston 231 relative to the seal 235 and the second barrel 219. The guides 263 further serve to secure the piston 231 to the second barrel 219 by means of a friction fit.

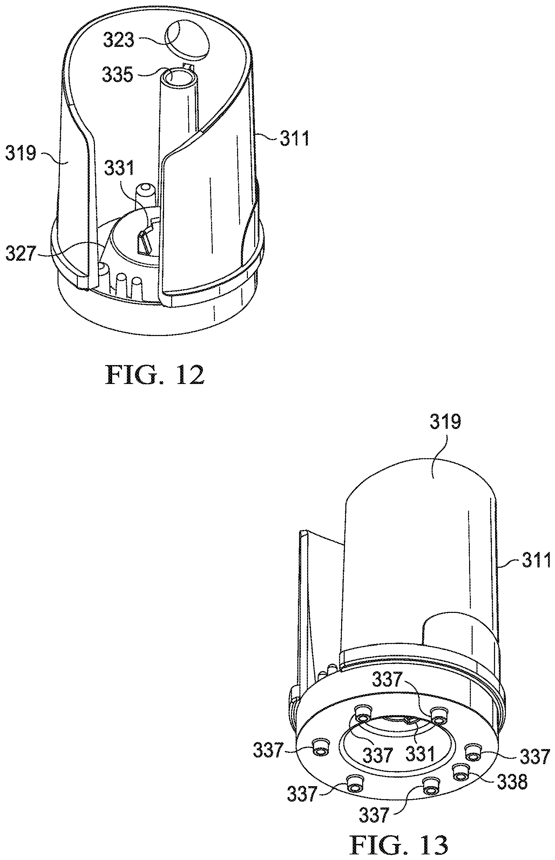

The piston 231 further includes an inner bowl 267 that is defined by the inner wall 251 and an inner floor 271. In some embodiments, the inner floor 271 may be two-tiered or multi-tiered as illustrated in FIG. 7, but the inner floor 271 may instead be single-tiered and/or substantially planar. The inner floor 271 may be positioned such that a recess 273 is defined beneath the inner floor 271 to receive an end of the piston spring 243 (see FIGS. 11 and 13). A regulator passage 275 passes through the inner floor 271 and may be seen in more detail in FIG. 7. A valve seat 279 may be positioned in the inner bowl 267 near the regulator passage 275 such that fluid communication through the regulator passage 275 may be selectively controlled by selective engagement of the valve seat 279 with a valve body (described in more detail with reference to FIG. 11).

A well 283 is positioned in the annulus 255 of the piston 231, and a channel 287 is fluidly connected between the well 283 and the inner bowl 267. The channel 287 allows fluid communication between the well 283 and the inner bowl 267.

FIG. 10 and FIG. 11 are perspective views illustrating additional details of the seal 235. The seal 235 includes a central portion 291 that is circumscribed by a skirt portion 295. A plurality of guidance apertures 299 are disposed in the central portion 291 to receive the guides 263 of the piston 231 when the reduced-pressure source 211 is assembled. A communication aperture 301 is similarly disposed in the central portion 291, and in some embodiments, the communication aperture 301 is radially spaced an equal distance from a center of the seal as the guidance apertures 299. The communication aperture 301 permits fluid communication through the central portion 291 of the seal 235 and with the well 283 of the piston 231 upon assembly.

The skirt portion 295 of the seal 235 extends axially and radially outward from the central portion 291. As illustrated in FIG. 7, the skirt portion 295 engages an inner surface 305 of the first barrel 215 to permit unidirectional fluid communication past the seal 235. The skirt portion 295 of the seal 235 may allow fluid to flow past the skirt portion 295 if the fluid flow is directed from the side of the seal 235 on which the piston 231 is disposed toward the opposite side of the seal 235. The skirt portion 295 may substantially prevent fluid flow in the opposite direction. While the skirt portion 295 of the seal 235 effectively controls fluid communication past the skirt portion 295, a valve member such as, for example, a check valve or other valve may be used to control fluid flow.

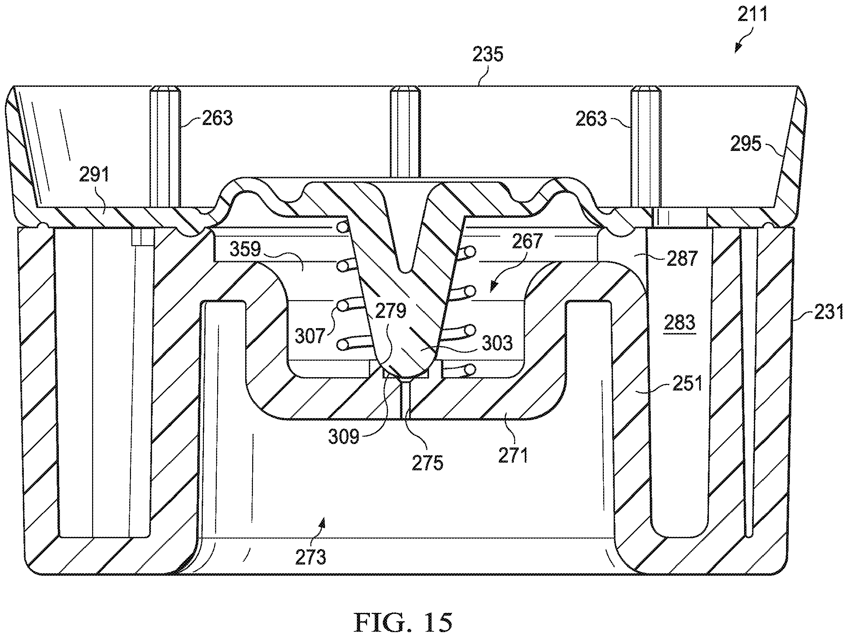

As illustrated in more detail in FIGS. 11 and 15, a valve body 303 is positioned on the central portion 291 of the seal 235. The valve body 303 may depend from the central portion 291 in an axial direction opposite the skirt portion 295. Although valve bodies of many types, shapes and sizes may be used, the valve body 303 may be cone-shaped with an apex 309 that is adapted to sealingly engage the valve seat 279 of the piston 231. While the valve body 303 is illustrated as being an integral part of the seal 235, the valve body 303 may be a separate component from the seal 235 that is provided to engage the valve seat 279.

In some embodiments, both the seal 235 and the valve body 303 are made from an elastomeric material, which could include without limitation a medical grade silicone. While many different materials may be used to construct, form, or otherwise create the seal 235 and the valve body 303, a flexible material may be used to improve the sealing properties of the skirt portion 295 with the inner surface 305 and the valve body 303 with the valve seat 279.

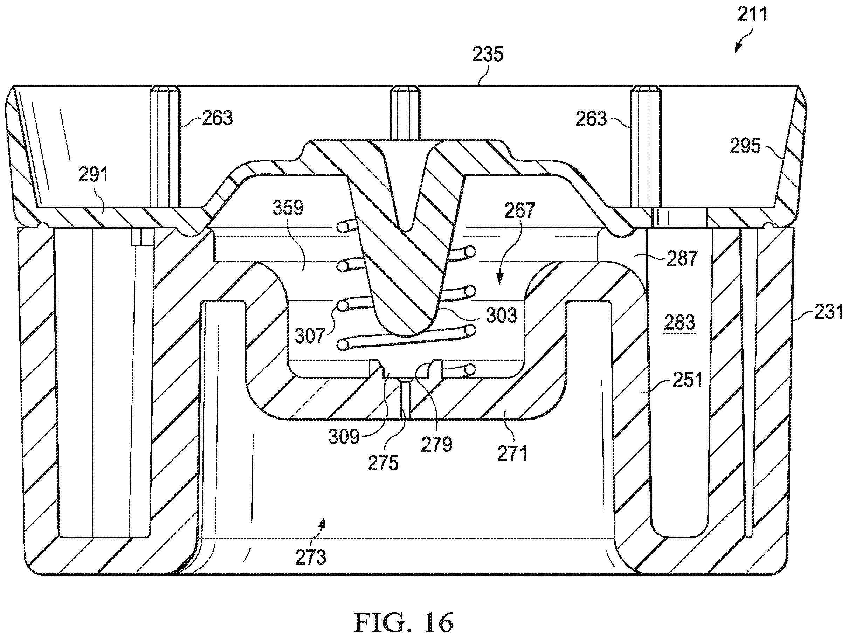

Referring more specifically to FIG. 7, a regulator spring 307 is provided to bias the valve body 303 away from the piston 231 and the valve seat 279. One end of the regulator spring 307 may be positioned concentrically around the valve seat 279 within the inner bowl 267 of the piston 231, while another end of the regulator spring 307 may be positioned around the valve body 303. The biasing force provided by the regulator spring 307 urges the valve body 303 toward an open position in which fluid communication is permitted through the regulator passage 275. FIG. 16 is a sectional view, illustrating additional details of the piston 231 and the seal 235. In the exemplary embodiment, if the spring 307 biases the valve body 303 toward the open position, only the central portion 291 of the seal 235 moves upward due to the flexibility of the seal.

As shown in FIGS. 9-11, the second barrel 219 includes a first housing portion 311 and a second housing portion 315. FIG. 12 is a perspective view, illustrating additional details of the first housing portion 311 of the second barrel 219. The first housing portion 311 includes an outer shell 319 having an aperture 323 disposed near an open end of the first housing portion 311. A floor 327 is integrally formed with or otherwise connected to the outer shell 319 on an end of the first housing portion 311 opposite the open end. A passage 331 may be centrally disposed in the floor 327. Referring to FIG. 5 and FIG. 7, a boss 333 is integrated with or connected to the first housing portion 311. The boss 333 includes the outlet port 227, which is physically aligned with the aperture 323 to allow a delivery tube to be fluidly connected to the outlet port 227. In some embodiments, the boss 333 is a ninety degree fluid fitting that permits the outlet port 227 to fluidly communicate with a conduit 335 positioned within the first housing portion 311. The conduit 335 may be a rigid conduit that is formed from the same or similar material to that of the outer shell, or in another embodiment, the conduit 335 may be flexible.

FIG. 13 is another perspective view, illustrating additional details of the first housing portion 311. The first housing portion 311 includes a plurality of guidance apertures 337 that are disposed in the floor 327 of the first housing portion 311. When the reduced-pressure source 211 is assembled, the guidance apertures 337 receive the guides 263 of the piston 231 to ensure that the second barrel 219 remains aligned with the piston 231. A friction fit between the guides 263 and the guidance apertures 337 assists in securing the relative positions of the piston 231 and the second barrel 219. It should be readily apparent, however, that the piston 231 and the second barrel 219 may be secured by other means. A communication aperture 338 is also disposed in the floor 327 to allow fluid communication with the conduit 335 through the floor 327.

Referring to FIGS. 9 and 10, the second housing portion 315 may include an end cap 339 integrally or otherwise connected to a guide 343. Together, the end cap 339 and the guide 343 slidingly engage the outer shell 319 of the first housing portion 311 to create a substantially closed second barrel 219 (with the exception of various apertures and passages described herein). While the second barrel 219 may be constructed from fewer components, the first housing portion 311 and the second housing portion 315 allows easier access within the second barrel 219 and also allows easier assembly of the reduced-pressure source 211. Additional advantages regarding the sliding engagement of the first housing portion 311 and the second housing portion 315 are explained in more detail below.

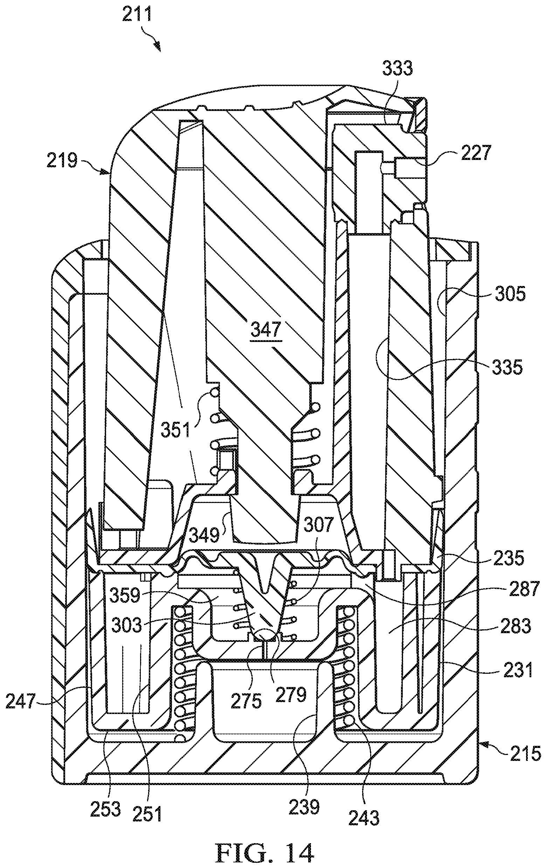

A shaft 347 extends from the end cap 339 and includes an engagement end 349 opposite the end cap 339. When the second barrel 219 is assembled, the shaft 347 may be substantially coaxial to a longitudinal axis of the second barrel 219 and extend through the passage 331 in the floor 327 of the first housing portion 311. A spring 351 is positioned within the second barrel 219 such that one end of the spring 351 bears upon the floor 327 of the first housing portion 311 and another end of the spring 351 bears upon the shaft 347 or another portion of the second housing portion 315. The spring 351 biases the shaft 347 and other portions of the second housing portion 315 toward a disengaged position (see position of the shaft 347 in FIG. 7) in which the engagement end 349 of the shaft 347 does not bear upon the seal 235 or the valve body 303. The sliding relationship and engagement between the first housing portion 311 and the second housing portion 315 allows a user to exert a force on the second housing portion 315 (against the biasing force of the spring 351) to move the second housing portion 315 to an engaged position. In the engaged position, the engagement end 349 of the shaft 347 bears upon the seal 235 above the valve body 303 (see FIG. 14), which forces the valve body 303 against the valve seat 279, thereby preventing fluid communication through the regulator passage 275.

When the reduced-pressure source 211 is assembled, as illustrated in FIG. 7, a charging chamber 355 is defined within the first barrel 215 beneath the piston 231. A regulated chamber 359 is defined within the inner bowl 267 of the piston 231 beneath the seal 235. The regulator passage 275 allows selective fluid communication between the charging chamber 355 and the regulated chamber 359 depending on the position of the valve body 303. The regulated chamber 359 fluidly communicates with the well 283 of the piston 231 through the channel 287. The well 283 is aligned with the communication aperture 301 of the seal 235 and the communication aperture 338 of the first housing portion 311, which allows fluid communication between the well 283 and the conduit 335 and the outlet port 227 of the second barrel 219.

While the regulator passage 275 is illustrated as being disposed within the piston 231, the regulator passage 275 could instead be routed through the wall of the first barrel 215. The regulator passage 275 could be any conduit that is suitable for allowing fluid communication between the regulated chamber 359 and the charging chamber 355.

In operation, the reduced-pressure source 211 is capable of being used with other components of a reduced pressure treatment system similar to those of reduced pressure treatment system 100 (see FIG. 1). The outlet port 227 of the reduced-pressure source 211 is adapted to be connected to a delivery tube or other conduit that is fluidly connected to a tissue site. Although a fluid canister could be integrated into the reduced-pressure source 211, in some embodiments, the reduced-pressure source 211 is not intended to collect wound exudates or other fluids within any internal chamber. In some embodiments, the reduced-pressure source 211 may either be used with low-exudating wounds, or an alternative collection system such as an external canister or absorptive dressing may be used to collect fluids.

Referring to FIG. 7, additional details of the reduced-pressure source 211 in the extended position are shown. To charge the reduced-pressure source 211, the second barrel 219 can be manually compressed into the first barrel 215 by a user such that the reduced-pressure source 211 is placed in a compressed position. Charging the reduced-pressure source 211 may also be referred to as priming the reduced-pressure source 211. FIG. 14 is a sectional view, illustrating additional details of the reduced-pressure source 211 in the compressed position. The force exerted by the user on the second barrel 219 to place the reduced-pressure source 211 in the compressed position of FIG. 14 must be greater than the biasing force provided by the piston spring 243. As the second barrel 219 compresses within the first barrel 215 and moves toward the closed end of the first barrel 215, the force being exerted on the second barrel 219 by the user is also transmitted to the seal 235 and the piston 231. The movement of the second barrel 219, the seal 235, and the piston 231 into the compressed position decreases the volume of the charging chamber 355. As the volume of the charging chamber 355 decreases, the pressure in the charging chamber 355 increases, but air and other gases within the charging chamber 355 are allowed to escape past the skirt portion 295 of the seal 235 due to the increased pressure within the charging chamber 355.

If the user releases the compressive force exerted upon the second barrel 219, the biasing force exerted by the piston spring 243 on the piston 231 moves the piston 231, the seal 235, and the second barrel 219 toward the extended position. As this movement occurs, the volume of the charging chamber 355 increases. Since the skirt portion 295 of the seal 235 allows only unidirectional flow, air and other gases are not permitted to enter the charging chamber 355 past the skirt portion 295. A resulting drop in pressure (i.e., a generation of reduced pressure) occurs within the charging chamber 355 as the volume increases. The amount of reduced pressure generated within the charging chamber 355 is dependent on the spring constant of the piston spring 243 and the integrity of the seal 235. In some embodiments, it is desired to generate a reduced pressure that is greater (i.e., a lower absolute pressure) than the therapy pressure to be supplied to the tissue site. For example, if it is desired to provide 125 mm Hg of reduced pressure to the tissue site, it may be desirable to have the charging chamber 355 charged to 150 mm Hg of reduced pressure.

The regulated chamber 359 provides the desired therapy pressure that is delivered to the outlet port 227 and the tissue site. If the reduced pressure within the charging chamber 355 is greater than the reduced pressure within the regulated chamber 359 and if the reduced pressure in the regulated chamber 359 is less than the desired therapy pressure, the upward force on the seal 235 (exerted by the increased absolute pressure in the regulated chamber 359 and the biasing force of the regulator spring 307, both against the atmospheric pressure exerted downward on the seal 235) moves the valve body 303 into the open position (see FIG. 16), thereby allowing fluid communication between the charging chamber 355 and the regulated chamber 359. The charging chamber 355 continues to charge the regulated chamber 359 with reduced pressure (i.e., the absolute pressure in the regulated chamber 359 continues to drop) until the reduced pressure in the regulated chamber 359, balanced against the atmospheric pressure above the seal 235, is sufficient to counteract the biasing force of the regulator spring 307 and move the valve body into the closed position. FIG. 15 is a sectional view, illustrating additional details of the seal 235 and the piston 231 in the closed position. If the regulated chamber 359 is charged with the desired therapy pressure, this pressure may be delivered to the outlet port 227 as detailed previously.

When the reduced-pressure source 211 is initially connected to a delivery tube and tissue site for treatment, it may be necessary to compress the second barrel 219 within the first barrel 215 multiple times. As each compression stroke is completed, the reduced pressure generated within the charging chamber 355 will pull air and any other gases from the delivery tube and the tissue site until the pressure within the tube and at the tissue site begins to approach the desired therapy pressure.

As the reduced-pressure source 211 is being primed by one or more compressions, it is important that air and other positively-pressurized gases being pushed out of the charging chamber 355 are pushed past the skirt portion 295 of the seal 235 and not into the regulated chamber 359. Positively pressurized gas flow to the regulated chamber 359 may transfer to the delivery tube and the tissue site, which would counteract the reduced pressure that is then being applied to the tissue site. To prevent positively pressurized gas from entering the regulated chamber 359, the shaft 347 is provided to engage the seal 235 and valve body 303. As the second barrel 219 is compressed within the first barrel 215, the second housing portion 315 moves relative to the first housing portion 311 so that the shaft 347 exerts a force on the valve body 303 that holds the valve body 303 in the closed position. Since the shaft 347 remains engaged during the entire compression, or charging stroke, of the reduced-pressure source 211, the air within the charging chamber 355 is vented past the seal 235 and not into the regulated chamber 359.

While the reduced-pressure source 211, including the first barrel 215, the second barrel 219, the piston 231, and the seal 235, have been described herein as being cylindrical, it will be readily apparent that all of these components may be other sizes or shapes. Additionally, the relative positions of the valve seat 279 and the valve body 303 may be reversed such that the valve body 303 is positioned below the valve seat 279.

If a dressing, delivery tube, or other component has a small leak, the valve body 303 can maintain a therapeutic pressure. For example, the regulated chamber 359 may be adapted to compensate for leaks that are less than about 1 L/min. However, the valve body 303 may not be able to maintain the therapy pressure if a leak exceeds such a limit, which is generally dependent upon the size of the restrictions on the entry and exit sides of the regulated chamber 359. In some embodiments, for example, where the charging chamber 335 is charged by an external reduced-pressure source, the regulated chamber 359 may compensate for a leak of about 1 L/min. In other embodiments, for example, where the charging chamber 335 is charged manually without the assistance of an external reduced-pressure source, the regulated chamber 359 may compensate for a leak of about several mL/hour.

The flow leaving regulated chamber 359 can be controlled by adjusting the bore size of regulator passage 275, and the flow coming into the regulated chamber 359 can be controlled by adjusting the size of the bore of a number of components in the fluid path, such as the conduit 112, tubing adapter 116, or outlet port 227. The size of the bores can be balanced such that a flow-induced drop in reduced pressure partially opens the valve body 303 if there is a leak in the dressing that exceeds a predetermined or configurable leak threshold, leaving a gap between the valve body 303 and the regulator passage 275. In some exemplary embodiments, the gap between the valve body 303 and the regulator passage 275 is less than about 0.1 mm. Optionally, the bore sizes can be balanced so that the valve body 303 remains open if no dressing is connected. Moreover, the bore sizes may be calibrated such that a flow of air through the gap produces an audible indicator, alerting an operator of an unexpected loss of therapeutic pressure. For example, a leak threshold flow rate may represent a leak flow rate that is sufficient to interfere with a prescribed therapy, and many applications may have a leak threshold of about 0.8 L/min. An audible indicator may be produced at the leak threshold if the regulator passage 275 is in the range of about 1 mm to about 1.5 mm and the conduit 112 has a lumen size of about 1.2 mm over a length of about 500 mm to 800 mm. The size of the gap (e.g., the distance between the apex 309 and the regulator passage 275) may be calibrated so that the pitch of the audible note changes as flow decreases or increases, thereby differentiating the size or rate of a leak.

Generally, if the lumen size of the conduit 112 is held constant at about 1.7 mm over a length of about 500 mm to 800 mm, a reduction in the size of the diameter of the regulator passage 275 may raise the leak threshold flow rate to initiate the audible warning. Similarly, if the size of the diameter of the regulator passage 275 is increased, the leak threshold flow rate to initiate the audible warning may be lowered. In some embodiments, the lumen size of the conduit 112 is about 1.7 mm and the diameter of the regulator passage 275 is about 0.7 mm; in response, the alarm threshold, the flow rate at which the audible warning may initiate, may be at approximately 1 L/min of flow. Although there may be large tolerances in the alarm threshold with the mechanical system described herein, flow between about 700 mL/min to about 1 L/min may cross the alarm threshold and result in an audible alert. Conventional electrical pump systems currently have an alarm triggered at a flow rate of approximately 1 L/min.

In other exemplary embodiments, the flow may be controlled with additional components, such as filters, which may include membranes, sintered porous materials, fibers, woven, or non-woven materials, for example. The valve body 303 and the regulator passage 275 may also be further designed to accentuate the audible feedback.

As illustrated in the example embodiments of FIGS. 11 and 21, the feedback module 404 may include the PCB 408 and the pressure sensor 412. The pressure sensor 412 may be positioned on the feedback module 404 to communicate with the charging chamber 355 within the first barrel 215. For example, the pressure sensor 412 may communicate with the charging chamber 335 through a conduit or an aperture, such as aperture 420 extending through the outer wall 416. In some embodiments, the aperture 420 can extend through the outer wall 416 from the charging chamber 355 to an interior of the housing extension 118. As illustrated in the example of FIG. 7, the aperture 420 may be coaxial with the piston spring 243 and the protrusion 239 in some embodiments. In other embodiments, the aperture 420 is not coaxial with the piston spring 243 and the protrusion 239.

The aperture 420 is configured to receive the pressure sensor 412 mounted on the PCB 408. Accordingly, the pressure sensor 412 may be in fluid communication with the interior of the charging chamber 355. Although only one pressure sensor 412 is shown, in other embodiments the feedback module 404 may include additional pressure sensors arranged to be received by additional apertures in fluid communication with the interior of the charging chamber 355. The pressure sensor 412 can sense the pressure within the charging chamber 355 and provide a signal indicating the sensed pressure to one or more components of the feedback module 404. A sealing member 422 may be disposed within the aperture 420, between respective surfaces of the outer wall 416 and the pressure sensor 412 in order to isolate the interior of the housing extension 118 from the interior of the charging chamber 355. Inserting the pressure sensor 412 into the aperture 420 in contact with the sealing member 422 seals the charging chamber 355 from the housing extension 118 and the atmosphere. Although the pressure sensor 412 is shown as having a generally cylindrical shape, other suitable shapes may be used. For example, the pressure sensor 412 may have a tapered shape to facilitate insertion through the sealing member 422 within aperture 420. Similarly, in other embodiments, inner walls of the sealing member 422 may be tapered to accommodate a tapered shape of the pressure sensor 412.

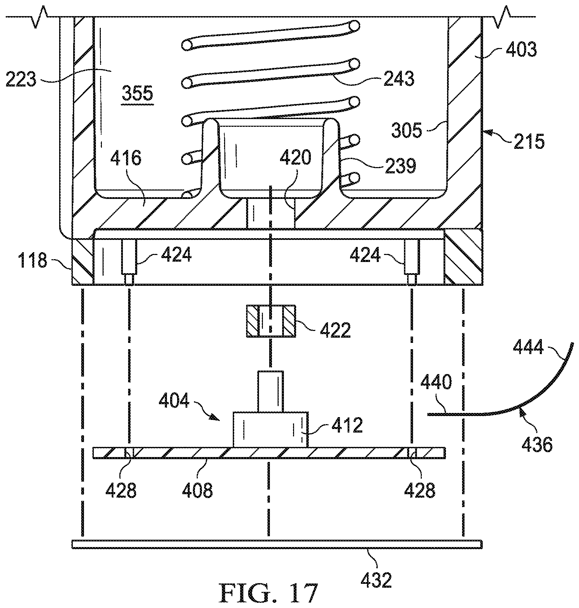

The outer wall 416 may include one or more mounting posts 424 extending therefrom, as illustrated in the examples of FIG. 7 and FIG. 17. For example only, the posts 424 may be integrally molded with the outer wall 416. The mounting posts 424 can be aligned with and insertably received by respective mounting openings 428 in the PCB 408 to retain the feedback module 404 within the housing extension 118. For example, the mounting posts 424 may retain the feedback module 404 within a module chamber 430, which may be defined in part by the housing extension 118 and a cover 432. As shown, a bottom surface of the PCB 408 may be coplanar with (i.e., flush) with a bottom surface of the housing extension 118. However, in other embodiments, the PCB 408 may be recessed with respect to the bottom surface of the housing extension 118 (i.e., not flush) so that the PCB 408 is closer to the outer wall 416.

The cover 432 may be provided to cover the bottom surface of the housing extension 118 and a bottom surface of the PCB 408 to enclose the feedback module 404 within the module chamber 430. The cover 432 may be, for example, a label printed with instructions for using the reduced-pressure source 211. Additionally, all components of the feedback module 404 may be arranged on an upper surface of the PCB 408 to facilitate adhesion of the cover 432 to the bottom surface of the PCB 408. The cover 432 also may be treated to provide a water resistant and/or splash resistant seal between the feedback module 404 and the environment external to the housing extension 118.

In some embodiments, a pull-tab 436 having a first end 440 and a second and 444 may be provided for initiating operation of the feedback module 404. The first end 440 of the pull-tab 436 may extend through a slot 448 between the housing extension 118 and the cover 432, and may be coupled to the feedback module 404 to electrically insulate internal contacts (not shown) of the feedback module 404 from each other to prevent power from being provided from a power source (e.g., a battery) to other components of the feedback module 404. The pull-tab 436 may be removed to allow electrical communication between the internal contacts and the power source to initiate operation of the feedback module 404. For example, a user may simply pull the second end 444 of the pull-tab 436 to remove the pull-tab 436 in order to initiate operation. In some embodiments, the second end 444 of the pull-tab 436 may be coupled to packaging used to enclose the reduced-pressure source 211. For example, the second end 444 may be coupled to the packaging by an adhesive such as, for example, an acrylic that is applied to an outward-facing surface of the second end 444. When the reduced-pressure source 211 is removed from the packaging, the second and 444 of the pull-tab 436 remains coupled to the packaging which removes the first end 440 of the pull-tab 436 from the slot 448 of the feedback module 404 to initiate operation of the feedback module 404. In some embodiments, the feedback module 404 may include a timer (not shown) that times out if the reduced-pressure source 211 is not used with in a recommended period of time. Accordingly, the user or caregiver may not be able to activate the feedback module 404 when removing the first end 440 of the pull-tab 436 in an attempt to use the reduced-pressure source 211 beyond a recommended usable period. The feedback module 404 also may include an audio output device that generates an audio indication that the feedback module 404 is powered on when the first end 440 of the pull-tap 436 is removed from the slot 448. The audio indication may provide a single tone or multiple escalating tones. The audio indication may be provided through the slot 448 when the first end 440 is removed from the slot 448.

In other embodiments, the feedback module 404 may be initiated using a button or other actuator. For example, the button may be arranged on the bottom surface of the PCB 408 under the cover 432 and may be pressed through the cover 432 or through an opening in the cover 432. The button may be used to initiate the feedback module 404 but does not provide a mechanism to deactivate the feedback module 404 once the feedback module 404 has been initiated. However, in some embodiments, the button may be used to silence or mute audio indicators from the feedback module 404.

In some embodiments, removing the cover 432 (e.g., after expiration of a usable period of the reduced-pressure source 211) may prevent further operation of the feedback module 404 and the reduced-pressure source 211. For example, removing the cover 432 may cause the PCB 408 to be separated from the housing extension 118, and/or may break one or more power traces on the PCB 408 to prevent further powering of components of the feedback module 404. For example, the cover 432 may be coupled to at least a portion of the PCB 408 (e.g., via a strong adhesive), causing the PCB 408 to be removed from the feedback module 404 when the cover 432 is removed. In other embodiments, one or more of the mounting posts 424 may be coupled to the respective mounting openings 428 using an adhesive and the power traces of the PCB 408 may be positioned proximate to one or more of the mounting openings 428. When the PCB 408 is removed and the mounting posts 424 are removed from the corresponding mounting openings 428, the power source may be disconnected to disable operation of the PCB 408 because the power trace is broken, cracked, separated from the PCB 408, or otherwise damaged. In yet another embodiment wherein the pressure sensor 412 is coupled to the PCB 408, the pressure sensor 412 may be removed from the sealing member 422 and the aperture 420 when the PCB 408 is removed from the PCB 408 that also renders the reduced-pressure source 211 inoperable.

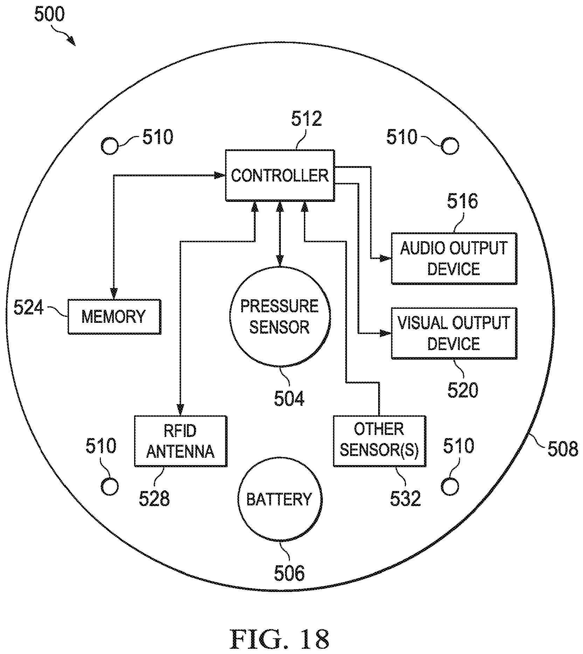

Referring now to FIG. 18, an electronic feedback module 500 is shown and may comprise a pressure sensor 504 that may be substantially similar to the pressure sensor 412 and a plurality of other components shown schematically as being mounted on a PCB 508 that are supported by mounting openings 510 and powered by a battery 506. These other components may comprise a controller 512, an audio output device 516, a visual output device 520 a memory device 524 and RFID antenna 528 all of which may be coupled directly or indirectly to the controller 512. The pressure sensor 504 may be configured to sense pressure within a chamber, such as the charging chamber 355, and can provide a pressure signal indicating the pressure sensed to the controller 512. The controller 512 can implement various functions of the feedback module 500 in response to signals received from the pressure sensor 504, or in response to one or more other sensed or stored parameters. For example, the controller 512 can implement these functions that may selectively activate the audio output device 516, such as a speaker, and/or the visual output device 520, such as one or more LEDs which may blink, flash, vary in brightness, generate different patterns or symbols. In some embodiments, the housing extension 118 may include an aperture or window (not shown) to facilitate communication of the audio or visual output. In some embodiments, a portion of the housing extension 118 may be transparent or opaque to allow light from the LEDs to be visible outside of the housing extension 118.

One of the functions of the controller 512 may include monitoring the pressure within the charging chamber 355 in response to the signal received from the pressure sensor 504 in order to determine whether the device needs to be recharged (e.g., the sensed pressure is less than a recharge threshold) and whether the sensed pressure is greater than an over-pressure threshold or is less than an under-pressure threshold. The recharge threshold may be set according to a pressure at which the charging chamber 355 will not be able to provide a desired reduced pressure (e.g., to the regulated chamber 359). For example, the recharge threshold may be set an offset amount above or below the desired reduced pressure (e.g., an offset between 50 and 100 mm Hg). The controller 512 may store the recharge threshold in the memory 524 as another function in response to a user or caregiver setting the desired reduced pressure for therapy treatments.

The controller 512 can control the audio output device 516 and the visual output device 520 based on the monitored pressure, which may be based, for example, on comparisons between the pressure signal and various parameters being calibrated such as the various threshold values stored in the memory 524. For example, if reduced pressure in the charging chamber 335 sensed by the pressure sensor 504 is less than the recharge threshold, the controller 512 can provide a feedback signal to activate the audio output device 516, which can provide an audible warning to the user. The audible warning may increase in volume as the sensed pressure continues to decrease if the user does not recharge the reduced-pressure source 211 within a predetermined period. Alternatively or additionally, the audible warning may be repeated periodically (e.g., at 1 or 5 minute intervals) until the reduced-pressure source 211 is recharged. Similarly, controller 512 may activate the visual output device 520 to provide a visible warning to the user.

Additionally or alternatively, the controller 512 may control the audio output device 516 and visual output device 520 based on a remaining usable period that the reduced-pressure source 211 is able to provide the desired reduced pressure set by the caregiver. For example, the reduced-pressure source 211 may have a predicted nominal life, which is stored as one or more nominal life indicators in the memory 524. The nominal life may be based on different parameters including, for example, a number of days (e.g., 30), a number of usage hours, or a number of times the reduced-pressure source 211 can be recharged. The controller 512 also may store one or more usage indications including, for example, how long the reduced-pressure source 211 has been powered on, how many hours the reduced-pressure source 211 has been used, or how many times the reduced-pressure source 211 was recharged. The controller 512 may then function to compare the usage indications to corresponding nominal life indicators indicating the predicted nominal life described above in order to determine a remaining useful life indicator. The controller 512 may selectively activate the audio output device 516 or the visual output device 520 in response to the remaining useful life indicator to provide an audible warning to the caregiver that the usefulness of the reduced-pressure source 211 is approaching or as close to expiring (e.g., when usage respective usage indications are within an offset of the corresponding nominal life indicators). The controller 512 may activate the audio output device 516 and/or the visual output device 520 to provide different warnings (e.g., different tones, frequencies, patterns, etc.) when the remaining useful life indicator has expired. The audible and/or visual warning indicating that the expiration is approaching and/or reached may be different than the audible and/or visual warning indicating that recharging is required. For example, the audible warning may be a different tone or pattern. Similarly, the visual warning may be a different pattern, frequency, or color.

The controller 512 may also determine whether the sensed pressure is greater than an over-pressure threshold or is less than the under-pressure threshold associated with the reduced-pressure source 211 and selectively activate the output devices 516 and 520 accordingly. For example, the over-pressure threshold may correspond to an offset amount greater than the recharge threshold. If the sensed pressure is greater than the over-pressure threshold, the controller 512 activates the audio output device 516 to provide an audible warning and/or may activate the visual output device 520 to provide a visual warning to the user. For example, the audible and visual warnings for the sensed pressure being greater than the over-pressure threshold may be the same as the audible and visual warnings for the expiration of the reduced-pressure source 211 being reached. Conversely, the under-pressure threshold may correspond to an offset amount less than the recharge threshold. If the sensed pressure is less than the over-pressure threshold, the controller 512 activates the audio output device 516 to provide an audible warning and/or may activate the visual output device 520 to provide a visual warning to the user. For example only, the audible and visual warnings for the sensed pressure being less than the under-pressure threshold may be the same as the audible and visual warnings for the expiration of the reduced-pressure source 211 being reached.

As described above, the warnings corresponding to the over-pressure threshold and the under-pressure threshold may indicate that the reduced-pressure source 211 is no longer functioning properly. Accordingly, the controller 512 may forego activating the warnings until the sensed pressure is greater than the over-pressure threshold or less than the under-pressure threshold for two or more successive measurements. In this manner, the controller 512 ensures that a single sensed pressure greater than the over-pressure threshold or less than the under-pressure threshold is not a temporary or anomalous condition caused by other operational factors.

To minimize battery usage and conserve power, the feedback module 500 may not monitor the pressure on a continual basis, but rather sample the pressure periodically such as, for example, once every one minute or five minutes. For example, the feedback module 500 may operate in a sleep mode (e.g., a mode where the components of the feedback module 500 are powered off or supplied a lower power level) for a predetermined period and transition to an awake mode to sample the pressure and respond accordingly, and then transition back to the sleep mode for the predetermined period. The controller 512 may transition the feedback module 500 between the sleep mode and the awake mode.

In some embodiments, the feedback module 500 may include an RFID antenna 528, one or more sensors 532, and/or other components to provide functions related to the operating of the reduced-pressure source 211. For example, the RFID antenna 528 may provide communication between the controller 512 and a user and/or device external to the reduced-pressure source. For example, the RFID antenna 528 may communicate data stored in the memory 524 to the user in response to queries. The data may include, but is not limited to, a time and date that the reduced-pressure source 211 was first activated (and/or how many days/hours ago the reduced-pressure source 211 was first activated), a total period the reduced-pressure source 211 has been at a desired reduced pressure, a total period the reduced-pressure source 211 has not been charged to the desired reduced pressure (and/or has been not charged at all), a number of times the reduced-pressure source 211 has been charged, an estimated remaining life of the reduced-pressure source 211, an estimated remaining power in the battery 506, etc.

The sensors 532 may include, but are not limited to, a thermal sensor, a motion and/or impact sensor, an accelerometer, etc. For example only, the thermal sensor may provide an indication of whether the patient has left a residence or other facility, whether the reduced-pressure source 211 has been stored in a location with temperature extremes that may have damaged the reduced-pressure source 211, etc. The motion and/or impact sensor may provide an indication of whether the reduced-pressure source 211 was dropped or damaged. The accelerometer may provide an indication of the ambulation and/or mobility of the patient.

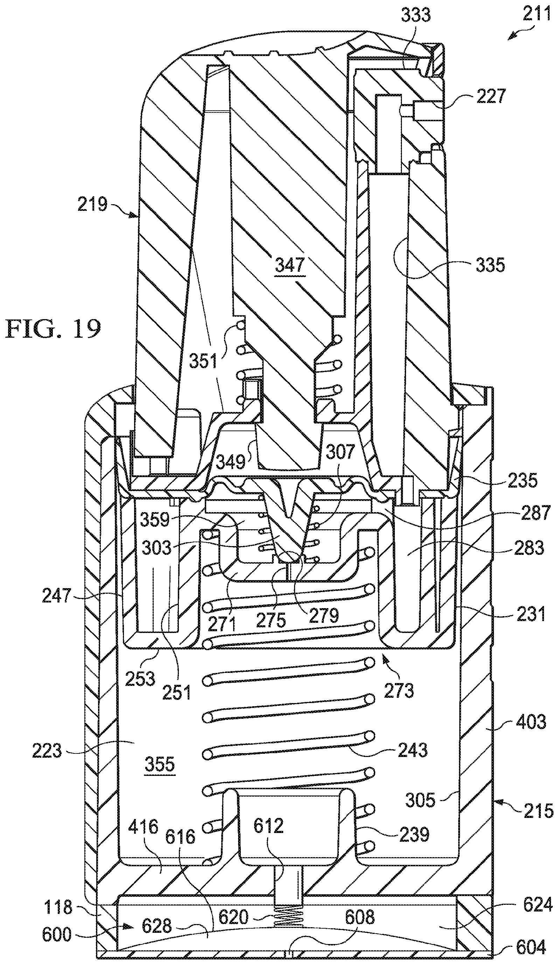

Referring now to FIG. 19, another embodiment of the reduced-pressure source 211 is shown, including the housing extension 118. In this embodiment, the reduced-pressure source 211 can provide audible feedback based on the pressure within the charging chamber 355, using mechanical components instead of (or, in some embodiments, in addition to) the electronic feedback module 500 described above. A pressure feedback chamber 600 is enclosed within the housing extension 118 using a cover 604. The cover 604 includes an aperture 608 configured to emit sound such as, for example, a whistling noise, when air passes through the aperture 608. For example, an aperture or conduit 612 fluidly connects the charging chamber 355 with the feedback chamber 600 and air passes through the aperture 608 based on the pressure within the charging chamber 355.

The feedback chamber 600 also includes a flexible member 616 and a biasing member such as, for example, a coil spring 620 that provides downward pressure against the flexible member 616. For example, the flexible member 616 may be a circular-shaped diaphragm circumferentially attached and sealed to an upper surface of the cover 604 and/or to an inner surface of the housing extension 118. Accordingly, a first portion 624 of the feedback chamber 600 above the flexible member 616 is sealed from atmosphere. Conversely, a second portion 628 of the feedback chamber 600 below the flexible member 616 is in fluid communication with atmosphere via the aperture 608.

When the charging chamber 355 is charged to a desired pressure, the resulting negative pressure in the charging chamber 355 draws the flexible member 616 upward against the spring member 620. Consequently, the spring member 620 is compressed and air is drawn into the second portion 628 through the aperture 608. Conversely, as the pressure in the charging chamber 355 deceases during use of the reduced-pressure source 211, the spring member 220 increasingly biases the flexible member 616 downward, forcing air from the second portion 628 through the aperture 608. The air being forced through the aperture 608 provides an audible warning that the reduced-pressure source 211 needs to be recharged. Parameters including, but not limited to, a volume of the second portion 628, a spring coefficient or force of the spring member 620, a material of the flexible member 616, a size and shape of the aperture 608, etc. can be selected to produce desired characteristics (e.g., frequency, duration, etc.) of the sound produced by the aperture 608.

In some embodiments, the flexible member 616 may be designed to deteriorate with use such that, subsequent to a predetermined number of recharges, a total usage period, etc., at least a portion of the flexible member 616 becomes unsealed (e.g., detached from the housing extension 118 or the cover 604) or otherwise allows fluid communication between the first portion 624 and the second portion 628. For example, a portion of the flexible member 616 that contacts the spring member 620 may wear over time until a leak develops. Accordingly, the reduced-pressure source 211 may become inoperable after a predetermined amount of time.

The systems, apparatuses, and methods described herein may provide significant advantages. For example, the feedback module 500 as described herein can provide to a user indications of various operating parameters of the reduced-pressure source 211. For example, the feedback module 500 can provide indications including, but not limited to, an indication of whether the reduced-pressure source 211 requires recharging and an indication of the remaining usable period of the reduced-pressure source 211. The reduced-pressure source 211 may further include a mechanism for separating the feedback module 500 from the reduced-pressure source 21 to facilitate disposal and prevent continued operation beyond the usable period. The feedback module 500 can also monitor, store, and provide various data regarding the operation of the reduced-pressure source 211 and certain behaviors of the user. The data may be indicative of whether the reduced-pressure source 211 was operated properly and as intended and may be used to inform subsequent treatment decisions.

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the controller 512 may be eliminated or separated from other components for manufacture, sale, or operation. In other example configurations, one or more of the components of the feedback module 500 may be combined into a single integrated circuit or system on a chip. The regulated chamber 359 may also be eliminated in some embodiments, or additional chambers may be incorporated.

## Claims

1. A manually actuated pump for providing reduced pressure treatment, comprising:
a housing defining a regulated chamber (359) and a charging chamber (355) in fluid communication with the regulated chamber (359), wherein the charging chamber (355) is configured to be supplied with a reduced pressure, and wherein the regulated chamber (359) is configured to regulate the reduced pressure to a regulated reduced pressure; and
a feedback module (500) comprising a controller (512), an output device (516), and a pressure sensor (504) in fluid communication with the charging chamber (355), wherein the pressure sensor (504) is configured to monitor reduced pressure within the charging chamber (355) and provide a pressure signal to the controller (512), and the controller (512) is configured to provide a feedback signal to the output device (516, 520) for generating at least one of an audible warning and a visual warning based on the pressure signal,
wherein the controller (512) is configured to activate at least one of an audio output device (516) and a visual output device (520) based on a comparison between the pressure signal and a recharge threshold.

2. The manually actuated pump of claim 1, further comprising:
a housing extension (118) coupled to the housing and enclosing the feedback module; and
a conduit (420) fluidly coupling the charging chamber (355) to the pressure sensor (504) through the housing.

3. The manually actuated pump
of claim 2, further comprising a sealing member (422) arranged in the conduit (420) between the pressure sensor (504) and an inner surface of the conduit.

4. The manually actuated pump
of claim 2, further comprising at least one mounting post (424) coupled to the housing, and wherein the feedback module (500) includes at least one mounting hole (510) arranged to receive the at least one mounting post (424).

5. The manually actuated pump
of claim 4, further comprising a cover (432) arranged over the housing extension (118) to define a module chamber (430) enclosing the feedback module (500).

6. The manually actuated pump
of claim 5, wherein the cover (432) is attached to a bottom surface of the feedback module (500).

7. The manually actuated pump
of claim 6, wherein the cover (432) is configured to cause the feedback module (500) to be removed if the cover (432) is removed.

8. The manually actuated pump
of claim 1, wherein the feedback module (500) includes a printed circuit board (508), and wherein the pressure sensor (504) is mounted on the printed circuit board (508).

9. The manually actuated pump
of claim 8, wherein the controller (512) is mounted on the printed circuit board (508).

10. The manually actuated pump
of claim 1, wherein the feedback module (500) includes memory (524) mounted on the printed circuit board (408) that stores the recharge threshold.

11. The manually actuated pump
of claim 10, wherein the memory (524) stores data indicative of a usable period of the pump, and wherein the controller (512) is configured to activate the at least one of the audio output device (516) and the visual output device (520) based on the data.

## Patentansprüche

1. Manuell betätigte Pumpe zum Bereitstellen einer Behandlung bei verringertem Druck, umfassend:
ein Gehäuse, das eine geregelte Kammer (359) und eine Beaufschlagungskammer (355) in Fluidverbindung mit der geregelten Kammer (359) definiert, wobei die Beaufschlagungskammer (355) konfiguriert ist, um mit einem verringerten Druck versorgt zu werden, und wobei die geregelte Kammer (359) konfiguriert ist, um den verringerten Druck auf einen geregelten verminderten Druck zu regeln; und
ein Rückkopplungsmodul (500), das eine Steuerung (512), eine Ausgabevorrichtung (516) und einen Drucksensor (504) in Fluidverbindung mit der Beaufschlagungskammer (355) umfasst, wobei der Drucksensor (504) konfiguriert ist, um verringerten Druck innerhalb der Beaufschlagungskammer (355) zu überwachen und um ein Drucksignal an die Steuerung (512) bereitzustellen, und die Steuerung (512) konfiguriert ist, um zum Erzeugen mindestens einer einer akustischen Warnung und einer visuellen Warnung basierend auf dem Drucksignal ein Rückkopplungssignal an die Ausgabevorrichtung (516, 520) bereitzustellen,
wobei die Steuerung (512) konfiguriert ist, um mindestens eine einer akustischen Ausgabevorrichtung (516) und einer visuellen Ausgabevorrichtung (520) basierend auf einem Vergleich zwischen dem Drucksignal und einem Wiederbeaufschlagungsschwellenwert zu aktivieren.

2. Manuell betätigte Pumpe nach Anspruch 1, weiter umfassend:
eine Gehäuseerweiterung (118), die an das Gehäuse gekoppelt ist und das Rückkopplungsmodul umschließt; und
eine Leitung (420), die die Beaufschlagungskammer (355) durch das Gehäuse fluidisch an den Drucksensor (504) koppelt.

3. Manuell betätigte Pumpe nach Anspruch 2, weiter umfassend ein Dichtelement (422), das in der Leitung (420) zwischen dem Drucksensor (504) und einer Innenfläche der Leitung angeordnet ist.

4. Manuell betätigte Pumpe nach Anspruch 2, weiter umfassend mindestens einen Montagepfosten (424), der an das Gehäuse gekoppelt ist, und wobei das Rückkopplungsmodul (500) mindestens eine Montageöffnung (510) beinhaltet, die angeordnet ist, um den mindestens einen Montagepfosten (424) aufzunehmen.

5. Manuell betätigte Pumpe nach Anspruch 4, weiter umfassend eine Abdeckung (432), die über der Gehäuseerweiterung (118) angeordnet ist, um eine Modulkammer (430) zu definieren, die das Rückkopplungsmodul (500) umschließt.

6. Manuell betätigte Pumpe nach Anspruch 5, wobei die Abdeckung (432) an einer Bodenfläche des Rückkopplungsmoduls (500) angebracht ist.

7. Manuell betätigte Pumpe nach Anspruch 6, wobei die Abdeckung (432) konfiguriert ist, um zu bewirken, dass das Rückkopplungsmodul (500) entfernt wird, wenn die Abdeckung (432) entfernt wird.

8. Manuell betätigte Pumpe nach Anspruch 1, wobei das Rückkopplungsmodul (500) eine Leiterplatte (508) beinhaltet und wobei der Drucksensor (504) an der Leiterplatte (508) montiert ist.

9. Manuell betätigte Pumpe nach Anspruch 8, wobei die Steuerung (512) an der Leiterplatte (508) montiert ist.

10. Manuell betätigte Pumpe nach Anspruch 1, wobei das Rückkopplungsmodul (500) einen an der Leiterplatte (408) montierten Speicher (524) beinhaltet, der den Wiederbeaufschlagungsschwellenwert speichert.

11. Manuell betätigte Pumpe nach Anspruch 10, wobei der Speicher (524) Daten speichert, die für einen Nutzungszeitraum der Pumpe indikativ sind, und wobei die Steuerung (512) konfiguriert ist, um die mindestens eine der akustischen Ausgabevorrichtung (516) und der visuellen Ausgabevorrichtung (520) basierend auf den Daten zu aktivieren.

## Revendications

1. Pompe actionnée manuellement pour fournir un traitement à pression réduite, comprenant :
un carter définissant une chambre régulée (359) et une chambre de charge (355) en communication de fluide avec la chambre régulée (359), dans laquelle la chambre de charge (355) est configurée pour être alimentée en pression réduite, et dans laquelle la chambre régulée (359) est configurée pour réguler la pression réduite en une pression réduite régulée ; et
un module de rétroaction (500) comprenant un mécanisme de commande (512), un dispositif de sortie (516), et un capteur de pression (504) en communication de fluide avec la chambre de charge (355), dans laquelle le capteur de pression (504) est configuré pour surveiller une pression réduite au sein de la chambre de charge (355) et fournir un signal de pression au mécanisme de commande (512), et le mécanisme de commande (512) est configuré pour fournir un signal de rétroaction au dispositif de sortie (516, 520) pour générer au moins l'un parmi un avertissement audible et un avertissement visuel sur la base du signal de pression,
dans laquelle le mécanisme de commande (512) est configuré pour activer au moins l'un parmi un dispositif de sortie audio (516) et un dispositif de sortie visuelle (520) sur la base d'une comparaison entre le signal de pression et un seuil de recharge.

2. Pompe actionnée manuellement selon la revendication 1, comprenant en outre :
une extension de carter (118) couplée au carter et enfermant le module de rétroaction ; et
un conduit (420) couplant de manière fluidique la chambre de charge (355) au capteur de pression (504) à travers le carter.

3. Pompe actionnée manuellement selon la revendication 2, comprenant en outre un élément d'étanchéité (422) agencé dans le conduit (420) entre le capteur de pression (504) et une surface interne du conduit.

4. Pompe actionnée manuellement selon la revendication 2, comprenant en outre au moins une tige de montage (424) couplée au carter, et dans laquelle le module de rétroaction (500) inclut au moins un trou de montage (510) agencé pour recevoir l'au moins une tige de montage (424).

5. Pompe actionnée manuellement selon la revendication 4, comprenant en outre un couvercle (432) agencé par-dessus l'extension de carter (118) pour définir une chambre de module (430) enfermant le module de rétroaction (500).

6. Pompe actionnée manuellement selon la revendication 5, dans laquelle le couvercle (432) est fixé à une surface inférieure du module de rétroaction (500).

7. Pompe actionnée manuellement selon la revendication 6, dans laquelle le couvercle (432) est configuré pour amener le module de rétroaction (500) à être retiré si le couvercle (432) est retiré.

8. Pompe actionnée manuellement selon la revendication 1, dans laquelle le module de rétroaction (500) inclut une carte de circuit imprimé (508), et dans laquelle le capteur de pression (504) est monté sur la carte de circuit imprimé (508).

9. Pompe actionnée manuellement selon la revendication 8, dans laquelle le mécanisme de commande (512) est monté sur la carte de circuit imprimé (508).

10. Pompe actionnée manuellement selon la revendication 1, dans laquelle le module de rétroaction (500) inclut une mémoire (524) montée sur la carte de circuit imprimé (408) qui mémorise le seuil de recharge.

11. Pompe actionnée manuellement selon la revendication 10, dans laquelle la mémoire (524) mémorise des données indiquant une période utilisable de la pompe, et dans laquelle le mécanisme de commande (512) est configuré pour activer l'au moins un parmi le dispositif de sortie audio (516) et le dispositif de sortie visuelle (520) sur la base des données.
